(19) Europäisches Patentamt
European Patent Office
Office européen des brevets

(11) EP 1 223 223 B1

(12) **EUROPÄISCHE PATENTSCHRIFT**

(45) Veröffentlichungstag und Bekanntmachung des Hinweises auf die Patenterteilung:
**17.05.2006 Patentblatt 2006/20**

(51) Int Cl.:
***C12P 41/00*** *(2006.01)*

(21) Anmeldenummer: **01131040.6**

(22) Anmeldetag: **28.12.2001**

(54) **Verfahren zur Herstellung von D- oder L-Menthol**

Method for preparing D- or L-menthol

Procédé pour préparer le D- ou le L-menthol

(84) Benannte Vertragsstaaten:
**AT BE CH CY DE DK ES FI FR GB GR IE IT LI LU MC NL PT SE TR**

(30) Priorität: **11.01.2001 DE 10100913**

(43) Veröffentlichungstag der Anmeldung:
**17.07.2002 Patentblatt 2002/29**

(73) Patentinhaber: **Symrise GmbH & Co. KG**
**37603 Holzminden (DE)**

(72) Erfinder:
- **Gatfield, Ian-Lucas, Dr.**
  **37671 Höxter (DE)**
- **Hilmer, Jens-Michael, Dr.**
  **37671 Höxter (DE)**
- **Bornscheuer, Uwe, Prof. Dr.**
  **17489 Greifswald (DE)**
- **Schmidt, Rolf, Prof. Dr.**
  **70329 Stuttgart (DE)**
- **Vorlova, Sandra**
  **70180 Stuttgart (DE)**

(74) Vertreter: **Eisenführ, Speiser & Partner**
**Patentanwälte Rechtsanwälte**
**Postfach 10 60 78**
**28060 Bremen (DE)**

(56) Entgegenhaltungen:
**EP-A- 0 196 625**

- **PERSICHETTI R A ET AL: "Candida Rugosa Lipase: Enantioselectivity Enhancements in Organic Solvents" TETRAHEDRON LETTERS, ELSEVIER SCIENCE PUBLISHERS, AMSTERDAM, NL, Bd. 37, Nr. 36, 2. September 1996 (1996-09-02), Seiten 6507-6510, XP004030729 ISSN: 0040-4039**
- **LANGRAND G. ET AL: 'Lipase Catalyzed Reactions and Strategy for Alcohol Resolution' TETRAHEDRON LETTERS Bd. 27, Nr. 1, 1986, UK, Seiten 29 - 32**
- **LALONDE J.J. ET AL: 'Cross-Linked Crystals of Candida rugosa Lipase: Highly Efficient Catalysts for the Resolution of Chiral Esters' JOURNAL OF THE AMERICAN CHEMICAL SOCIETY Bd. 117, 1995, Seiten 6845 - 6852, XP002041053**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 223 223 B1

## Beschreibung

Die Erfindung betrifft ein Verfahren zur Herstellung von D- oder L-Menthol durch enantioselektive enzymatische Spaltung von D,L-Menthylderivaten.

Verfahren zur synthetischen Herstellung von Menthol sind allgemein bekannt (Common Fragrance and Flavor Materials; Bauer, K., Garbe, D: und Surburg, H., Verlag VCH, Weinheim, 1990, 2. Ausgabe, S. 44-46). Sind die so erhaltenen Produkte racemische Gemische, so sind sie im Geschmack und Geruch dem natürlich vorkommenden L-Menthol z. B. aus Pfefferminzöl deutlich unterlegen. Daher besteht ein starkes Interesse an Trennverfahren für D,L-Menthol.

Man kann die Trennung beispielsweise mit Hilfe von physikalischen Verfahren erreichen. Solche Verfahren umfassen z. B. die fraktionierte Kristallisation der Salze optisch aktiver Amine mit racemischem Menthylhydrogenphthalat oder -hydrogensuccinat. Ferner kann D- oder L-Menthol aus racemischen Mentholgemischen abgetrennt werden, indem man das Gemisch mit einer optisch aktiven Säure, z.B. Menthoxyessigsäure, verestert und das Gemisch diastereomerer Verbindungen durch Kristallisation abtrennt. Das D- bzw. L-Menthol erhält man durch die Verseifung des diastereomeren Esters.

Ein weiteres großtechnisch angewandtes Verfahren (DE-A 2 109 456) zur Abtrennung von optisch reinem D- und L-Menthol aus D,L-Mentholgemischen läuft über einen Carbonsäurementhylester als Zwischenstufe. Es werden bevorzugt die Ester der Benzoesäure oder der Hexahydrobenzoesäure, ferner die Ester der 4-Methylbenzoesäure, der 3,5-Dinitrobenzoesäure und der 4-Ethoxybenzoesäure verwendet. Bei dem Verfahren handelt es sich um die selektive Kristallisation optischer Antipoden, die in so einer hohen Reinheit erhalten werden, dass die Weiterverarbeitung ohne weitere Reinigungsoperationen durchgeführt werden kann.

Außerdem kann mit Hilfe von Enzymen bzw. Mikroorganismen L-Menthol aus D,L-Mentholgemischen isoliert werden.

Es ist auch bekannt, dass Lipasen Ester in wässrigen Medien hydrolysieren und eine hohe Spezifität und Selektivität besitzen können. Außerdem haben in bestimmten organischen Lösungsmitteln einige Lipasen die Fähigkeit, die Rückreaktion zu katalysieren, um Ester aus den entsprechenden Säuren und Alkoholen zu synthetisieren.

Verschiedene Strategien zur Gewinnung von reinem L-Menthol aus dem racemischen D,L-Mentholgemisch sind angewandt worden. So ist z. B. aus Tetrahedron Letters, 27, (1986) 29 bekannt, dass die Lipase von Candida rugosa aus einem racemischem Menthyllaurat bevorzugt das L-Menthol (ee: 70 %) durch Hydrolyse in wässrigem Medium freisetzt. Diese enantioselektive Bevorzugung wurde ebenfalls bei der Veresterung von racemischem Menthol mit Laurinsäure gezeigt, wobei das L-Menthyllaurat mit hoher enantiomerer Reinheit (ee: 86 %) gebildet wird. In nichtwässrigem Medium kann mit Lipase racemisches Menthol enantioselektiv mit Laurinsäure verestert werden, wobei wiederum das L-Menthyllaurat bevorzugt gebildet wurde (ee: 95 %). Diese Umsetzung ist nach 10 Stunden praktisch abgeschlossen. Die Umesterung von D,L-Menthol mit Trilaurin bzw. D,L-Menthyllaurat mit Isobutanol verläuft mit ähnlich hoher Enantioselektivität, ist aber extrem langsam (Reaktionsdauer: 15 Tage und länger).

Es ist auch bekannt, Umsetzungen unter Enzymkatalyse in nicht wässrigen Medien durchzuführen, wenn die Substanzen in Wasser nur schlecht löslich sind. Als Alternative zu organischen Lösungsmitteln bieten sich überkritische Fluide und im speziellen überkritisches Kohlendioxid an. So auch ist dies für die Racemattrennung von D,L-Menthol aus Chemie Ingenieur Technik, 69, (1986) 29 bekannt und zwar durch die enantioselektive Umesterung von verschiedenen Acetaten mit racemischem Menthol. Die besten Ergebnisse werden mit dem Enolester Isopropenylacetat erzielt. Solche Ester haben den Vorzug, dass nach erfolgter Umsetzung der durch die Hydrolyse gebildete Alkohol - in diesem Falle Isopropenylalkohol - sofort in das entsprechende Keton isomerisiert und daher für eine etwaige Rückreaktion nicht zur Verfügung steht. Die untersuchten Enzyme sind die Lipase AY aus Candida rugosa, die Lipase PS aus Burkholderia cepacia (ehemals Pseudomonas cepacia), das Novozym 435 aus Candida antarctica B, das Lipozym IM 60 aus Rhizomucor miehei und die Esterase EP 10 aus Pseudomonas marginata.

Die Esterase EP 10 kann aus rekombinanten E. coli-Stämmen, die das Gen für die EP 10 Esterase enthalten, gewonnen werden. Die Esterase EP 10 zeigt die weitaus höchsten Enantioselektivitäten im System. Das Novozym 435 zeigt unter den gewählten Bedingungen so gut wie keinen Umsatz bei der Umesterung mit den verschiedenen Acetaten.

Die Enantioselektivität der Lipase aus Candida rugosa (Lipase AY) gegenüber racemischem Menthol lässt sich nach den Angaben in Biotechnol. Prog. 11, (1995) 270 durch die gezielte Behandlung der Lipase mit nichtionischen Tensiden signifikant erhöhen. Aus diesen Untersuchungen geht eindeutig hervor, dass die Effektivität der Veresterung von L-Menthol mit Laurinsäure in organischem Medium sehr vom Enzym abhängt. Die Lipase aus Candida rugosa ist eindeutig effektiver bei dieser Umsetzung als die Lipasen aus Rhizopus sp., Burkholderia cepacia, Pseudomonas sp., Mucor javanicus, Aspergillus niger bzw. aus Schweinepankreas. Zusätzlich wird gezeigt, dass durch die Behandlung mit nichtionischen Tensiden die Effektivität der Lipase aus Candida rugosa auf etwa das Fünffache steigt.

Aus Tetrahedron Letters 39, (1998) 4333 ist bekannt, dass die Verwendung von Mikrowellenbestrahlung im Falle der Schweinepankreaslipase zu keiner Änderung der Reaktionsgeschwindigkeit oder der Enantioselektivität bei der Veresterung des racemischen Menthols mit Palmitinsäure führt.

Lipasen sind ebenfalls in der Lage, Carbonsäureanhydride als Acyl-Donor anzunehmen. Carbonsäureanhydride haben, wie im Falle der Enolester schon angesprochen wurde, den Vorteil, dass der Acyltransfer quasi irreversibel ist. Nach Enzyme and Microbial Technology 18, (1996) 536 ist die Lipase AY-30 aus Candida rugosa befähigt, eine gewisse Enantioselektivität bei der Umsetzung von racemischem Menthol mit Essigsäureanhydrid, Proplonsäureanhydrid und Buttersäureanhydrid auszuüben. Die besten Ergebnisse mit diesem Enzym werden mit Buttersäureanhydrid nach 48 Stunden in n-Hexan als Lösungsmittel erzielt (ee: 86 % des gebildeten L-Menthylbutyrates).

Die Enantioselektivität der Umsetzung ist wohl von der verwendeten Lipase bzw. vom Anhydrid stark abhängig. So ist aus Microbiol. Biotechnol 43, (1995) 639 bekannt, dass die Lipase OF 360 aus Candida rugosa und Propionsäureanhydrid eine recht hohe optische Reinheit des gebildeten L-Menthylpropionates (ee: 95 %) aufweist.

Eine weitere Möglichkeit, L-Menthol aus D,L-Menthol-Gemischen herzustellen, besteht darin, racemische Estermischungen enzymatisch enantioselektiv zu spalten. So ist aus Dechema Biotechnol. Conf. (1989) 141 bekannt, D,L-Menthylacetat mit der Lipase aus Candida rugosa in einer Hydrolyse umzusetzen, wobei das freigesetzte L-Menthol auf eine recht geringe Enantioselektivität des Enzyms hindeutet.

Schließlich ist aus LALONDE J. J. et al., "Cross-Linked Crystals of Candida rugosa Lipase: Highly Efficient Catalysts for the Resolution of Chiral Esters", Journal of the American Chemical Society, 1995, 117, 6845-6852 bekannt, zur Herstellung von (-)-Menthol Menthylacetat enzymatisch mit einer immobilisierten Lipase von *Candida rugosa* zu spalten.

Aufgabe der vorliegenden Erfindung ist es, ein für die technische Anwendung geeignetes D,L-Menthol bzw. deren Derivate mit hoher absoluter Enantioselektivität zu spalten, um reines L-Menthol oder D-Menthol oder einen reinen L-Menthylester oder D-Menthylester zu erhalten.

Es wurde ein Verfahren zur Herstellung von D- oder L-Menthol gefunden, dadurch gekennzeichnet, dass D,L-Menthylderivate enantioselektiv enzymatisch durch rekombinante Lipase 1 aus Candida rugosa gespalten werden.

Nach dem erfindungsgemäßen Verfahren erhält man überraschenderweise die Enantiomere mit einem Enantiomerenüberschuss (ee-Wert) von über 99 % und einer Selektivität (E-Wert) von > 100.

D,L-Menthylderivate für das erfindungsgemäße Verfahren sind Verbindungen der Formel

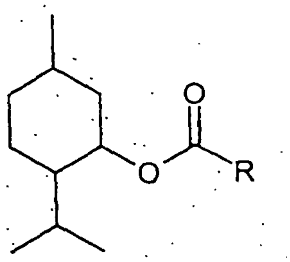

worin

R Wasserstoff, geradkettiges oder verzweigtes $C_1$-$C_{20}$- Alkyl, $C_3$-$C_8$-Cycloalkyl, $C_6$-$C_{14}$-Aryl, $C_7$-$C_{15}$-Arylalkyl, $C_1$-$C_{20}$-Alkoxy, $C_1$-$C_{20}$-Alkylamino bedeutet, wobei die zuvor genannten Kohlenwasserstoffreste gegebenenfalls ein- oder mehrfach substituiert sein können mit Hydroxy, Formyl, Oxy, $C_1$-$C_6$-Alkoxy, Carboxy, Mercapto, Sulfo, Amino, $C_1$-$C_6$-Alkylamino oder Nitro der Halogen, bevorzugt Chlor.

Bevorzugte D,L-Menthylderivate sind Ester des D,L-Menthols mit aliphatischen oder aromatischen Carbonsäuren. Beispielsweise seien die folgenden Ester genannt:

D,L-Menthylacetat, D,L-Menthylbenzoat, D,L-Menthylisovalerianat.

Insbesondere bevorzugt wird D,L-Menthylbenzoat.

Die D,L-Menthylderivate für das erfindungsgemäße Verfahren sind an sich bekannt.

Es ist bekannt, dass Lipasen auch durch rekombinante DNA-Techniken gewonnen werden können (EP A 238 023). Dabei wird das für die Lipase codierende Gen von einem ausgewählten Stamm durch dem Fachmann bekannte Methoden in einen Empfängerorganismus transferiert. Dieser Empfängerorganismus produziert die Lipase.

In einer besonders bevorzugten Ausführung werden rekombinante Lipasen, die auf einem Trägermaterial immobilisiert sind, verwendet. Geeignete Trägermaterialien sind beispielsweise Kunststoffe wie Polypropylen, Polystyrol, Polyvinylchlorid, Polyurethan, Polyacryl, Latex, Nylon oder Teflon, Polysaccaride wie Agarose oder Dextran, Ionenaustauscherharze (sowohl kationische wie anionische), Silicon-Polymere wie beispielsweise Siloxane oder Silicate, beispielsweise Glas. Immobilisierungstechniken für Enzyme sind dem Fachmann bekannt (K. Mosbach, "Immobilized Enzymes", *Methods in Enzymology* 44, Academic Press, New York, 1976) und beinhalten cross-linking, Adsorption oder kovalente Bindung an das Trägermaterial.

Lipasen aus *Candida rugosa* werden auch kommerziell vertrieben, beispielsweise Lipase AY (Vertreiber: Amano, Nagoya, Japan) .

Überraschenderweise wurde in einer bevorzugten Form der vorliegenden Erfindung festgestellt, dass die Hydrolyse von D,L-Menthylbenzoat mit rekombinanter Lipase aus Candida rugosa (WO 99/14338) mit sehr hoher Enantioselektivität (E > 100) und einem Enantiomerenüberschuss an (-)-Menthol von > 99,9 % abläuft. Dieses Ergebnis wurde durch gaschromatographische Analyse, NMR-Spektroskopie und durch Polarimetrie bestätigt.

Das unterschiedliche hydrolytische Verhalten der beiden Candida rugosa Lipasen (kommerzielle und rekombinante) kann damit erklärt werden, dass kommerzielle Präparationen nicht nur das gewünschte Enzym, sondern eine ganze Reihe von Isoenzymen mit etwas unterschiedlichen Eigenschaften enthalten können. Durch SDS-PAGE Untersuchungen konnte gezeigt werden, dass die verwendete rekombinante Lipase nur eine Proteinbande aufweist (s. WO 99/14338), während die Lipase AY mehrere Proteinbanden aufweist.

Üblicherweise können als Lösungsmittel für das erfindungsgemäße Verfahren Wasser, wässrige Puffer und organische Lösungsmittel eingesetzt werden. Als organische Lösungsmittel werden bevorzugt Hexan, Cyclohexan, Heptan, Cycloheptan, Toluol, Dichlormethan, Acetonitril, Dimethylformamid, Dioxan, Tetrahydrofuran oder Ethanol verwendet. Als wässrige Puffer werden bevorzugt Phosphat- oder Acetatpuffer verwendet.

Für das erfindungsgemäße Verfahren setzt man im allgemeinen 1 bis 10000 Units (U), bevorzugt 10 bis 1000 Units (U) der Lipase bezogen auf 0,01 mmol des Menthylderivates ein.

Die Spaltung nach dem erfindungsgemäßen Verfahren wird im allgemeinen im Temperaturbereich von 0 bis 90°C, bevorzugt von 20 bis 60°C durchgeführt.

Die Spaltung nach dem erfindungsgemäßen Verfahren wird im allgemeinen im pH-Bereich von 1 bis 12, bevorzugt bei etwa pH 7, durchgeführt.

Das erfindungsgemäße Verfahren kann beispielsweise wie folgt durchgeführt werden:

> In einem ersten Schritt wird in einem Fermenter das Enzym analog WO 99/14338 produziert (vgl. Beispiel 1). In einem zweiten Schritt wird die so erhaltene Lipase gereinigt (vgl. Beispiel 1). In einem dritten Schritt erfolgt die enantioselektive enzymatische Spaltung des Menthylderivats (vgl. Beispiel 3)

Die so hergestellten reinen Menthol-Enantiomeren genügen hohen analytischen und sensorischen Anforderungen.

## Beispiele

### Beispiel 1

### Fed-Batch-Fermentation *Pichia pastoris*

| | |
|---|---|
| Vektor: | pGAP (Invitrogen) |
| Plasmid: | Lip 1 (Lipase aus Candida rugosa) |
| Expression: | konstitutiv |

### Fermentation

Die Fed-Batch-Fermentation wurde in einem 42 1 Bioreaktor (Bioengineering) bei 30°C und pH 6 in komplexem Medium durchgeführt. Das Medium enthielt 1 % Hefe-Extrakt, 2 % Pepton, 1 % Glycerin und 0,1 M K-Phosphat Puffer pH 6. Die Feeding-Lösungen bestanden für die reine Glucosefütterung aus 20 % Glucose, bei der Mischfütterung aus 20 % Glucose / 5 % Glycerin. Der Bioreaktor wurde mit 500 ml einer Übernacht-Schüttelkultur mit einer $OD_{600}$ von 2 bis 3 in obengenanntem Medium inokkuliert. Die Rührerdrehzahl betrug 400 UpM, die Belüftungsrate 15 Nl/min. Während der Fermentation wurden die optische Dichte bei 600 nm, die Biofeuchtmasse (BFM), die Biotrockenmasse (BTM), sowie die lipolytische Aktivität des Überstandes bestimmt. Die Fütterung erfolgte zum ersten Mal nach 24 Stunden, ab dann alle 12 Stunden je 300 bis 600 ml der Feedinglösung. Beginn der Mischfütterung war bei 72 Stunden. Die Fermentation wurde nach 170 bis 190 Stunden beendet.

Die Aktivität betrug nach untenstehender Aufarbeitung 40 000 U/g gefriergetrocknetem Konzentrat. Die Gesamtausbeute waren 21 g Trockensubstanz.

**Aufreinigung der in komplexen Medium kultivierten CRL (*Candida rugosa* Lipase)**

Trotz der Sekretion der reifen *Candida rugosa* Lipase in aktiver Form durch *Pichia pastoris* in das Medium, ergab die Analyse der angefertigten SDS-Gele, dass im Überstand noch kontaminierende Proteine enthalten waren. Daher wurde ein Reinigungsprotokoll für die Aufreinigung der rekombinanten Lipase entwickelt.

Nach einer Cross-Flow-Filtration (Sartorius, Göttingen, Sartocon Cassette: 0,2 μm) wurden 50 ml des Fermentationsüberstands dialysiert (Dialyseschlauch Spectra/Por® ), um die den nächsten Reinigungsschritt störenden Salze zu entfernen. Anschließend wurde die Lipaselösung durch Ultrafiltration mit einer 30 kD Membran (Pall, Omega Minisette, MG: 30.000) weiter eingeengt. Die FPLC-Säule wurde mit DEAE-Sepharose gepackt, die Säule mit 25 mM Tris-HCl Puffer (pH 7,5) äquilibriert und die Lipaselösung aufgetragen. Nach einem Waschschritt mit dem Äquilibrierungspuffer wurde die Lipase mit einem NaCl-Gradienten eluiert. Die Fraktionen wurden mit dem pNPP-Schnelltest (s. u.) auf Lipaseaktivität überprüft. Jene Fraktionen, die eine Gelbfärbung aufwiesen, wurden vereinigt, ultrafiltriert, lyophylisiert (Finn Aqua Lyovac GT2) und die lypolytische Aktivität am pH-Stat bestimmt. Das Reinigungsprotokoll ist in Tabelle 1 zusammengefasst.

## Tabelle 1

**Reinigungstabelle für CRL aus Kultivierungsüberstand von *Pichia pastoris* in komplexem Medium**

| Volumen der Lipaselösung [ml] | Reinigungsschritt | Gesamtaktivität [U] * | spez. Aktivität [U mg$^{-1}$] | Gesamt-Ausbeute (Ausbeute je Reinigungsschritt) [%] |
|---|---|---|---|---|
| 50 | - | 213 500 | 52 | - |
| 270 | Dialyse | 197 100 | 146 | 92 (92) |
| 37 | Ultrafiltration | 185 000 | 166 | 87 (94) |
| 50 | Ionenaustausch-chromatographie mit DEAE-Sepharose | 89 600 | 5978 | 42 (49) |

*** Aktivitäten wurden am pH-stat gegen Tributyrin gemessen**

**Enzymassay**

Die Aktivität wurde routinemäßig mit einem pH-Stat (Metrohm) und pH 7,2 bestimmt.

66 mM Tributyrin werden mit 20 mg/ml Gummiarabicum als Stabilisator, emulgiert und mit dem Ultraturrax (T25, Janke & Kunkel) für 7 min. bei maximaler Geschwindigkeit homogenisiert.

20 ml der Assaylösung wurden vorgelegt und mit 10 bis 100 μl der Enzymlösung versetzt. Dann wurde die Aktivität mit dem pH-Stat bestimmt. Ein Unit wurde als die Menge an Enzym, die ein μmol Fettsäure pro Minute freisetzt, bezeichnet.

**pNPP-Schnelltest**

Um schnell eine große Menge an Proben testen zu können, wird ein Schnelltest verwendet. Lösung A besteht aus p-Nitrophenylpalmitat (pNPP, 10 mM) in Isopropanol gelöst. Lösung B aus Tris-Puffer (100 mM, pH 7,5), Cholat (0,8 % (w/v)) und Gummi Arabicum (1 % (w/v)). Die Reaktionsmischung setzt sich aus 9 Teilen Lösung B und einem Teil Lösung A zusammen und muss immer frisch angesetzt werden. Die zu untersuchende Lösung (50 μl) wurde in Mikrotiterplatten pipettiert und die Reaktionsmischung (200 μl) zugegeben. Die durch die Spaltung des Substrats entstehende gelbe Farbe wurde entweder visuell abgeschätzt oder spektrometrisch quantifiziert.

Beispiel 2

**Vorversuche zur Extraktion des Menthylbenzoats**

Die Hydrolyse von D,L-Menthylbenzoat wurde in Natriumphosphat Puffer (pH 7,2, 100 mM) mit Gummi arabicum (0,2 % (m/v)) als Lösungsvermittler durchgeführt. Anschließend musste das Reaktionsgemisch für die gaschromatographische Analytik mit einem geeigneten Lösungsmittel extrahiert werden.

Zur Ermittlung eines geeigneten Lösungsmittels wurden äquimolare Mengen an D,L-Menthylbenzoat und D, L-Menthol in Isooktan gelöst, um einen Standard des Verhältnisses der Signalflächen [Menthylbenzoat/Menthol] zu erhalten. Die gaschromatographische Bestimmung ergab ein Signalflächenverhältnis [Menthylbenzoat/Menthol] von 1,7.

Anschließend wurde das Lösungsmittel am Rotationsverdampfer entfernt, der Rückstand in 10 ml Natriumphosphat Puffer (pH 7,2, 100 mM) aufgenommen, mit Gummi arabicum (0,2 % (m/v)) versetzt, 10 min. homogenisiert und je 1 ml in Kunststoffreaktiongefäße aliquotiert. Nach einstündiger Inkubation bei 40°C wurde das Gemisch mit unterschiedlichen Lösungsmitteln überschichtet (je 500 µl) und 1 Std. unter Schütteln extrahiert. Tabelle 2 zeigt die nach GC Messungen erhaltenen Verhältnisse der Signalflächen [Menthylbenzoat/Menthol].

Tabelle 2 Lösungsmittel zur Extraktion

| Lösungsmittel | Signalflächen [Menthylbenzoat/Menthol] |
|---|---|
| Isooktan | 0,2 |
| Hexan | 0,1 |
| Toluol | 1,4 |
| Chloroform | 1,2 |
| Essigsäureethylester | 1,7 |
| Diethylether | 1,1 |
| Isopropylether | 1,4 |

Essigsäureethylester erwies sich somit als am besten geeignetes Lösungsmittel für die Extraktion von Menthylbenzoat und Menthol.

Beispiel 3

**Hydrolyse von D,L-Menthylbenzoat mit rekombinanter Lipase aus *Candida rugosa* (rek. CRL)**

Die Reaktion wurde in je 1 ml Natriumphosphat Puffer (pH 7,2, 100 mM) bei 40°C mit 0,2 % (m/v) Gummi arabicum als Lösungsvermittler durchgeführt. Die eingesetzte Enzymmenge betrug jeweils 400 U aufgereinigte *Candida rugosa* Lipase auf 0,01 mmol D,L-Menthylbenzoat. Die Enantiomerenüberschüsse an Menthol wurden gaschromatographisch bestimmt, die des Menthylbenzoats anhand der Signalflächen berechnet.

Tabelle 3 Hydrolyse von D,L-Menthylbenzoat mit rek. CRL

| **Reaktionszeit [h]** | **Enantiomerenüberschuss** | | **Umsatz** | **Enantioselektivität** |
|---|---|---|---|---|
| | **[%ee$_S$]** | **[%ee$_P$]** | **[%]** | |
| Umsetzung bei 40°C | | | | |
| 2 | 2 | >99 | 2 | >100 |
| 4 | 11 | >99 | 10 | >100 |
| 6 | 28 | >99 | 22 | >100 |
| 8 | 82 | >99 | 45 | >100 |

Aus Tabelle 3 geht hervor, dass die rekombinante Lipase aus *Candida rugosa* eine sehr hohe Enantioselektivität (E > 100) bezüglich der Hydrolyse von D,L-Menthylbenzoat unter den gewählten Reaktionsbedingungen zeigt.

Beispiel 4

**Bestimmung des Temperaturoptimums für die Hydrolyse von D,L-Menthylbenzoat**

In der Tabelle 4 sind die Ergebnisse der Hydrolyse von D,L-Menthylbenzoat bei unterschiedlichen Temperaturen zusammengefasst. Die Umsetzungen wurden in je 1 ml Natriumphosphat Puffer (pH 7,2, 100 mM) mit 0,2 %(m/v) Gummi arabicum als Lösungsvermittler durchgeführt. Zur Ermittlung des Temperaturoptimums wurden Reaktionstemperaturen von 30°, 40°, 50° und 60°C gewählt. Die eingesetzte Enzymmenge betrug jeweils 800 U aufgereinigte *Candida rugosa* Lipase auf 0,01 mmol D,L-Menthylbenzoat. Die Enantiomerenüberschüsse an Menthol wurden gaschromatographisch bestimmt, die des Menthylbenzoats berechnet.

Tabelle 4 Hydrolyse von D,L-Menthylbenzoat mit rek. CRL

| | **Enantiomerenüberschuss** | | **Umsatz Enantioselektivität** | |
|---|---|---|---|---|
| **Reaktionszeit [h]** | **[%$ee_S$]** | **[%$ee_P$]** | **[%]** | |
| Umsetzung bei 30°C | | | | |
| 2 | 19 | >99 | 16 | >100 |
| 4 | 30 | >99 | 23 | >100 |
| 6 | 37 | >99 | 27 | >100 |
| 8 | 42 | >99 | 30 | >100 |
| Umsetzung bei 40°C | | | | |
| 2 | 22 | >99 | 18 | >100 |
| 4 | 35 | >99 | 26 | >100 |
| 6 | 45 | >99 | 31 | >100 |
| 8 | 84 | >99 | 46 | >100 |
| Umsetzung bei 50°C | | | | |
| 2 | 63 | >99 | 39 | >100 |
| 4 | 99 | >99 | 50 | >100 |
| Umsetzung bei 60°C | | | | |
| 2 | 69 | >99 | 41 | >100 |
| 4 | 99 | >99 | 50 | >100 |

Aus Tabelle 4 ergibt sich ein Temperaturoptimum für die Hydrolyse von D,L-Menthylbenzoat von 50°C. Bei der Umsetzung bei 60°C wurde keine weitere Reaktivitätssteigerung beobachtet. Bei beiden Temperaturen ergab sich bereits nach 4 Std. unter den verwendeten Reaktionsbedingungen ein Umsatz von 50 %, und damit die vollständige Umsetzung des gewünschten Menthylbenzoatenantiomers.

Beispiel 5

**Vergleich der rekombinanten *Candida rugosa* Lipase mit kommerziell erhältlichen Lipasen**

Die bisherigen Versuche zur Hydrolyse von D,L-Menthylbenzoat wurden mit der am Institut für Technische Biochemie, Universität Stuttgart, rekombinant hergestellten und aufgereinigten Lipase aus *Candida rugosa* durchgeführt (Brocca 1998).

Ferner wurden folgende kommerziell erhältliche Lipasen eingehender auf ihre Stereoselektivität gegenüber racemischem Menthylbenzoat untersucht: kommerzielle Lipasen aus *Candida rugosa* (Amano AY), *Burkholderia cepacia* (ehemals *Pseudomonas cepacia;* Roche Diagnostics, Penzberg; Chirazyme L-1), *Rhyzomucor miehei* (Roche Diagnostics, Penzberg; Chirazyme L-9) und *Rhizopus oryzae* (Amano F). Tabelle 5. zeigt das Ergebnis der Umsetzung von D, L-Menthylbenzoat mit der Lipase aus *Rhizomucor miehei* bei 40°C und einer Reaktionszeit von 16 Std. Der Enantiomerenüberschuss an Produkt beträgt lediglich 2 %, was auf eine äußerst geringe Enantioselektivität schließen lässt.

Tabelle 5 Hydrolyse von D,L-Menthylbenzoat mit RML

| | Enantiomerenüberschuss | | Umsatz [%] | Enantioselektivität |
|---|---|---|---|---|
| | [%$ee_S$] | [%$ee_P$] | | |
| *Rhizomucor miehei* Lipase | n.b. | 2 | 2 | 2 |

Die Lipasen aus *Burkholderia cepacia* und *Rhizopus oryzae* zeigten nach 16 Std. Reaktionszeit keinen Umsatz.

Tabelle 6 zeigt die Hydrolyse von D,L-Menthylbenzoat mit der von Amano Pharmaceutical Co., Ltd., Nagoya Japan kommerziell erhältlichen *Candida rugosa* Lipase. Die Umsetzungen wurden bei 40° und 50°C durchgeführt.

Tabelle 6 Hydrolyse von D,L-Menthylbenzoat mit kommerzieller CRL (Amano AY)

| Reaktionszeit [h] | Enantiomerenüberschuss | Umsatz | Enantioselektivität |
|---|---|---|---|
| | [%$ee_P$] | [%] | |
| Umsetzung bei 40°C | | | |
| 2 | 70 | 20 | 7 |
| 4 | 69 | 65 | |
| 6 | 69 | 78 | |
| 8 | 68 | 91 | |
| Umsetzung bei 50°C | | | |
| 2 | 69 | 47 | 10 |
| 4 | 59 | 50 | 7 |

Aus Tabelle 6 geht hervor, dass die kommerzielle *Candida rugosa* Lipase zwar eine hohe Aktivität, aber auch eine sehr geringe Enantioselektivität gegenüber Menthylbenzoat aufweist.

Beispiel 6

**Hydrolyse von D,L-Menthylbenzoat mit rekombinanter *Candida rugosa* Lipase - Präparativer Ansatz**

*Reaktionsgleichung:*

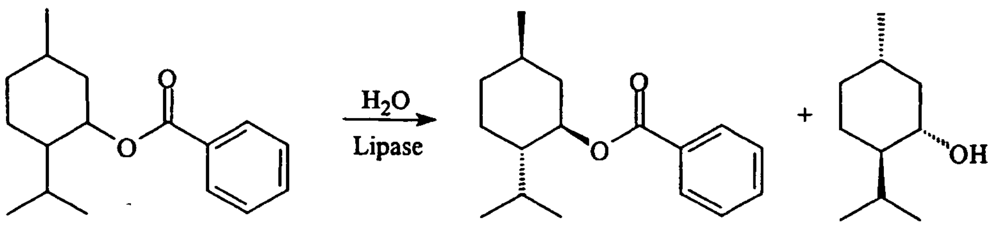


*rac*-Menthylbenzoat (+)-Menthylbenzoat (-)-Menthol

*Durchführung:*

2,1 g (8 mmol) D,L-Menthylbenzoat wurde in einem 500 ml-Rundkolben in 250 ml Natriumphosphat Puffer (pH 7,0, 100 mM) suspendiert und 5 g rekombinante gereinigte *Candida rugosa* Lipase zugegeben. Die Reaktion wurde 20 h bei 40°C unter starkem Rühren durchgeführt. Der Reaktionsverlauf wurde mit Dünnschichtchromatographie verfolgt. Anschließend wurde das Reaktionsgemisch mit Toluol extrahiert (250 ml, 2 x 100 ml), die vereinigten organischen

Phasen über $Na_2SO_4$ getrocknet und am Rotationsverdampfer eingeengt.

Das verbleibende, leicht gelbliche Reaktionsgemisch wurde mit Hilfe der Säulenchromatographie (Kieselgel) gereinigt. Als Laufmittel wurde Petrolether/Essigsäureethylester im Verhältnis 10:1 verwendet.

*Ausbeute:*

Es ergab sich eine Ausbeute von 255 mg (1,6 mmol, 20,0 %) an (-)-Menthol und 421 mg (1,6 mmol, 20,0 %) an Menthylbenzoat.

*Charakterisierung:*

**Drehwertbestimmung:**

Menthol:

$[\alpha]_D^{20}$ = - 51,3 (c = 1.00, $CH_2Cl_2$) $[\alpha]_D^{20}$ = -50 ± 1

Menthylbenzoat:

$[\alpha]_D^{20}$ = + 86,3 (c = 1.00, $CH_2Cl_2$) $[\alpha]_D^{20}$ = + 84,5 (c = 1.00, $CH_2Cl_2$)

**NMR-Spektroskopie:**

Menthol

$^1$H-NMR ($CDCl_3$, 500.1 MHz) δ gegen TMS: 0.81 (d; J =6.9; 3H); 0.92 (2d; 6 H); 0.97 (d, 1H); 1.10 (d; J = 10.2; 1H); 1.40-1.47 (m; 2H); 1.59-1.67 (m; 2H); 1.96 (d, 1H); 2.17 (m; 1H); 3.42 (dt; J = 10.4,4.1; 1H).

C-NMR ($CDCl_3$, 125.8 MHz) δ gegen TMS: 16.10; 21.03; 22.23; 23.15; 25.83; 31.66; 34.56; 45.06; 50.15; 71.54.

Menthylbenzoat:

$^1$H-NMR ($CDCl_3$, 500.1 MHz) δ gegen TMS: 0,80 (d; J = 7.0; 3H); 0.92 (2d; J = 5.2, 4.7; 6H); 1.08-1.19 (m; 2H);1.53-1.58 (m; 2H);1.70-1.75 (m; 2H); 1.93-2.00 (m; 1H); 2.11-2.15 (m; 1H); 4.94 (dt; J = 10.9, 4.4; 1H); 7.41-7.56 (m; 3H); 8,04 (t; 2H).

$^{13}$C-NMR ($CDCl_3$, 125.8 MHz) δ gegen TMS: 16.52;20.78; 22.05;23.64; 26.50; 31.45; 34.34; 40.98; 47.29; 74.82; 128.29; 129.56; 130.88; 132.68; 166.09.

**Gaschromatographie:**

Menthol: ≥ 99,9 %ee

Beispiel 7

**Hydrolyse von D,L-Menthylacetat**

In der Tabelle 7 sind die Ergebnisse der Hydrolyse von D,L-Menthylacetat zusammengefasst. Die Umsetzungen wurden in Natriumphosphat Puffer (pH 7,2, 100 mM) mit 0,2 %(m/v) Gummi arabicum als Lösungsvermittler durchgeführt. Zur Ermittlung des Temperaturoptimums wurden Reaktionstemperaturen von 40°, 50° und 60°C gewählt. Die eingesetzte Enzymmenge betrug jeweils 800 U aufgereinigte *Candida rugosa* Lipase auf 0,01mmol D,L-Menthylacetat. Die Enantiomerenüberschüsse wurden gaschromatographisch bestimmt.

Tabelle 7 Hydrolyse von D,L-Menthylacetat mit rek. CRL (ITB)

| Reaktionszeit [h] | Enantiomerenüberschuss | | Umsatz | Enantioselektivität |
|---|---|---|---|---|
| | [%$ee_S$] | [%$ee_P$] | [%] | |
| Umsetzung bei 40°C | | | | |
| 2 | 54 | >99 | 35 | >100 |
| 4 | 78 | >99 | 44 | >100 |
| 6 | 89 | >99 | 47 | >100 |
| 8 | 93 | >99 | 48 | >100 |
| Umsetzung bei 50°C | | | | |
| 2 | 74 | >99 | 43 | >100 |
| 4 | 93 | >99 | 48 | >100 |
| 6 | 98 | >99 | 49 | >100 |
| 8 | 98 | >99 | 50 | >100 |
| Umsetzung bei 60°C | | | | |
| 2 | 84 | >99 | 46 | >100 |
| 4 | 92 | >99 | 48 | >100 |
| 6 | 96 | >99 | 49 | >100 |
| 8 | 96 | >99 | 49 | >100 |

Aus Tabelle 7 geht hervor, dass die rekombinante Lipase aus *Candida rugosa* hohe Enantioselektivität bezüglich der Hydrolyse von D,L-Menthylacetat unter den gewählten Reaktionsbedingungen zeigt.

Vergleichend wurde die Hydrolyse von D,L-Menthylacetat mit der von Amano Pharmaceutical Co., Ltd., Nagoya Japan, kommerziell erhältlichen Lipase aus *Candida rugosa* (Amano AY) durchgeführt (Tabelle 8). Aus der Tabelle geht deutlich hervor, dass diese kommerzielle Präparation eine deutlich geringere Enantioselektivität als die rekombinante *Candida rugosa* Lipase gegenüber D,L-Menthylacetat aufweist.

Tabelle 8 Hydrolyse von D,L-Menthylacetat mit kommerzieller CRL (Amano AY)

| Reaktionszeit [h] | Enantiomerenüberschuss | | Umsatz | Enantioselektivität |
|---|---|---|---|---|
| | [%$ee_S$] | [%$ee_P$] | [%] | |
| Umsetzung bei 40°C | | | | |
| 6 | 84 | 48 | 53 | 7 |
| 14 | 76 | 33 | 67 | 4 |

Beispiel 8

**Hydrolyse von D,L-Menthylisovalerianat**

In der Tabelle 9 sind die Ergebnisse der Hydrolyse von D,L-Menthylisovalerianat zusammengefasst. Die Umsetzungen wurden in Natriumphosphat Puffer (pH 7,2, 100 mM) mit 0,2 % (m/v) Gummi arabicum als Lösungsvermittler durchgeführt. Zur Ermittlung des Temperaturoptimums wurden Reaktionstemperaturen von 40°, 50° und 60°C gewählt. Die eingesetzte Enzymmenge betrug jeweils 800 U aufgereinigte *Candida rugosa* Lipase auf 0,01 mmol D,L-Menthylisovalerianat. Die Enantiomerenüberschüsse wurden gaschromatographisch bestimmt.

Tabelle 9 Hydrolyse von D,L-Menthylisovalerianat mit rek. CRL (ITB)

| Reaktionszeit [h] | Enantiomerenüberschuss | | Umsatz | Enantioselektivität |
|---|---|---|---|---|
| | [%$ee_S$] | [%$ee_P$] | [%] | |
| Umsetzung bei 40°C | | | | |
| 2 | 5 | >99 | 5 | >100 |
| 4 | 12 | >99 | 11 | >100 |
| 6 | 22 | >99 | 18 | >100 |
| 8 | 31 | >99 | 24 | >100 |
| | | | | |
| Umsetzung bei 50°C | | | | |
| 2 | 9 | >99 | 8 | >100 |
| 4 | 20 | >99 | 17 | >100 |
| 6 | 40 | >99 | 29 | >100 |
| 8 | 63 | >99 | 39 | >100 |
| | | | | |
| Umsetzung bei 60°C | | | | |
| 2 | 10 | >99 | 9 | >100 |
| 4 | 20 | >99 | 17 | >100 |
| 6 | 30 | >99 | 23 | >100 |
| 8 | 31 | >99 | 24 | >100 |

Aus Tabelle 9 geht hervor, dass bei der Hydrolyse von D,L-Menthylisovalerianat mit der rekombinanten *Candida rugosa* Lipase Enantiomerenüberschüsse des Produktes von >99 %ee erreicht wurden. Das Temperaturoptimum für diese Reaktion konnte bei 50°C festgestellt werden. Bei 60°C ist nach 6 Std. ein Aktivitätsverlust des Enzyms zu erkennen, da der Umsatz stagniert, was auf die Denaturierung des Enzyms bei hohen Temperaturen zurückzuführen sein dürfte.

Beispiel 9

**Hydrolyse von D,L-Menthylanthranilat**

Die Hydrolyse von D,L-Menthylanthranilat ergab unter den gewählten Standardbedingungen (Natriumphosphat Puffer pH 7,2, 100 mM, 30°, 40°, 50° und 60°C, Reaktionszeit 24 Std. keinen Umsatz.

Beispiel 10

Immobilisierung der freien Lipase aus *Candida rugosa*

Bestimmung des geeigneten Trägermaterials

Für die Immobilisierung der aufgereinigten, nativen Lipase aus *Candida rugosa* wurden unterschiedliche Trägermaterialien getestet. Die Immobilisierung auf Celite® 545 (Fluka), EP100 (Polypropylen Pulver 200 - 400 micron, Akzo Nobel), Hyflo Super Cell® (Fluka), $SiO_2$ (Fluka) und $Al_2O_3$ (Fluka) beruht auf hydrophobe Adsorption der Lipase, während die Bindung an DEAE-Sepharose (Pharmacia Biotech) auf ionische Wechselwirkungen beruht. Je nach Trägermaterial wurden zwischen 2000 und 3000 Units pro 1 g Träger an aufgereinigter Lipase verwendet.

Nach Durchführung der Immobilisierung wurden die Proben filtriert und die Aktivitäten im Filtrat und Immobilisat am pH-Stat (pH 7,2, 30°C) gegen Tributyrin gemessen. Tabelle 10 zeigt die Effizienz der Immobilisierung auf den unterschiedlichen Trägermaterialien.

Tabelle 10

Aktivität nach Immobilisierung im Filtrat und des Immobilisats.

| Trägermaterial | Aktivität im Filtrat [%] | Aktivität Immobilisat [%] |
|---|---|---|
| **Celite** | 91 | 4 |
| **EP100** | 16 | 43 |
| **Hyflo** | 64 | 8 |
| **$SiO_2$** | 48 | 20 |
| **$Al_2O_3$** | - | - |
| **DEAE** | 69 | 7 |

Da mit den Trägermaterialien EP100 und $SiO_2$ die höchsten Ausbeuten an aktiv immobilisierter *Candida rugosa* Lipase erzielt wurden, wurden diese Immobilisate in der Hydrolyse von D,L-Menthylbenzoat eingesetzt.

Beispiel 11

**Hydrolyse von D,L-Menthylbenzoat mit rekombinanter *Candida rugosa* Lipase immobilisiert auf EP100 und $SiO_2$**

Die Reaktion wurde in je 15 ml Natriumphosphat Puffer (pH 7,2, 100 mM) bei 50°C mit 0,2 % (m/v) Gummi arabicum als Lösungsvermittler durchgeführt. Die eingesetzte Menge an Immobilisat betrug jeweils 1200 Units auf 0,1 mmol D,L-Menthylbenzoat. Die Reaktionsdauer betrug 8 Std. Die Enantiomerenüberschüsse wurden gaschromatographisch bestimmt.

Tabelle 11 Hydrolyse von D,L-Menthylbenzoat mit immobilisierter rek. CRL

| Trägermaterial | Enantiomerenüberschuss [%$ee_P$] | Umsatz [%] | Enantioselektivität |
|---|---|---|---|
| EP100 | >99 | 43 | >100 |
| $SiO_2$ | >99 | 45 | >100 |

Aus Tabelle 11 geht hervor, dass die immobilisierte Lipase aus *Candida rugosa* eine sehr hohe Enantioselektivität (E > 100) bezüglich der Hydrolyse von D,L-Menthylbenzoat zeigt. Dieses Ergebnis entspricht den mit der freien Lipase aus *Candida rugosa* erhaltenen Ergebnissen.

**Patentansprüche**

1. Verfahren zur Herstellung von D- oder L-Menthol, wobei durch rekombinante Lipase 1 aus *Candida rugosa* in einem wässrigen Medium eine Substanz der allgemeinen Formel

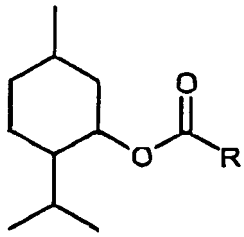

gespalten wird, worin

R Wasserstoff, ein geradkettiges oder verzweigtes $C_1$-$C_{20}$- Alkyl, $C_3$-$C_8$-Cycloalkyl, $C_6$-$C_{14}$-Aryl, $C_7$-$C_{15}$-Arylalkyl, $C_1$-$C_{20}$-Alkoxy oder $C_1$-$C_{20}$-Alkylamino bedeutet, wobei die zuvor genannten Kohlenwasserstoffreste gegebenenfalls ein- oder mehrfach substituiert sein können mit Hydroxy, Formyl, Oxy, $C_1$-$C_6$-Alkoxy, Carboxy, Mercapto, Sulfo, Amino, $C_1$-$C_6$-Alkylamino oder Nitro oder Halogen.

**2.** Verfahren nach Anspruch 1, wobei die eingesetzte Substanz ein aliphatischer oder aromatischer Menthylester ist.

**3.** Verfahren nach einem der Ansprüche 1 bis 2, wobei die eingesetzte Substanz Menthylbenzoat ist.

**4.** Verfahren nach einem der Ansprüche 1 bis 3, **dadurch gekennzeichnet, dass** die Spaltung bei etwa pH 7 und einer Temperatur von 10 bis 70°C durchgeführt wird.

**5.** Verfahren nach einem der Ansprüche 1 bis 4, wobei die Lipase immobilisiert ist.

## Claims

**1.** A process for the production of D- or L-menthol, wherein a substance of the general formula

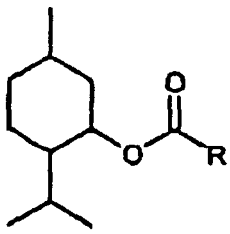

is cleaved by recombinant lipase 1 from *Candida rugosa* in an aqueous medium, in which substance

R means hydrogen, a linear or branched $C_1$-$C_{20}$ alkyl, $C_3$-$C_8$ cycloalkyl, $C_6$-$C_{14}$ aryl, $C_7$-$C_{15}$ arylalkyl, $C_1$-$C_{20}$ alkoxy or $C_1$-$C_{20}$alkylamino, wherein the above-stated hydrocarbon residues may optionally be mono- or polysubstituted with hydroxy, formyl, oxy, $C_1$-$C_6$ alkoxy, carboxy, mercapto, sulfo, amino, $C_1$-$C_6$ alkylamino or nitro or halogen.

**2.** A process according to claim 1, wherein the substance used is an aliphatic or aromatic menthyl ester.

**3.** A process according to either one of claims 1 to 2, wherein the substance used is menthyl benzoate.

**4.** A process according to any one of claims 1 to 3, **characterised in that** cleavage is performed at around pH 7 and a temperature of 10 to 70°C.

**5.** A process according to any one of claims 1 to 4, wherein the lipase is immobilised.

## Revendications

**1.** Procédé pour la préparation de D- ou L-menthol, dans lequel on dissocie par recombinaison de lipase 1 provenant de *Candida rugosa* dans un milieu aqueux une substance de la formule générale

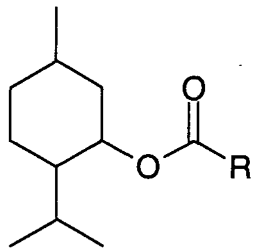

dans laquelle

R représente un atome d'hydrogène, un groupe alkyle en $C_1$-$C_{20}$ linéaire ou ramifié, cycloalkyle en $C_3$-$C_8$, aryle en $C_6$-$C_{14}$, arylalkyle en $C_7$-$C_{15}$, alcoxy en $C_1$-$C_{20}$ ou alkylamino en $C_1$-$C_{20}$, les restes hydrocarbonés cités précédemment pouvant être éventuellement substitués une ou plusieurs fois avec un groupe hydroxy, formyle, oxy, alcoxy en $C_1$-$C_8$, carboxy, mercapto, sulfo, amino, alkylamino en $C_1$-$C_6$ ou nitro ou un atome d'halogène.

**2.** Procédé selon la revendication 1, dans lequel la substance utilisée est un ester menthylique aliphatique ou aromatique.

**3.** Procédé selon l'une quelconque des revendications 1 à 2, dans lequel la substance utilisée est le benzoate de menthyle.

**4.** Procédé selon l'une quelconque des revendications 1 à 3, **caractérisé en ce que** la dissociation est réalisée à un pH d'environ 7 et à une température de 10 à 70°C.

**5.** Procédé selon l'une quelconque des revendications 1 à 4, dans lequel la lipase est immobilisée.